# EUROPEAN PATENT SPECIFICATION

(11) **EP 3 679 082 B1**
(45) Date of publication and mention of the grant of the patent: **01.11.2023**
(21) Application number: 18778566.2
(22) Date of filing: 04.09.2018
(51) Int. Cl.: C07C 37/52, C08G 64/30

(54) **A METHOD OF RECOVERING PHENOL IN A MELT POLYCARBONATE POLYMERIZATION**
VERFAHREN ZUR RÜCKGEWINNUNG VON PHENOL IN EINER SCHMELZEPOLYCARBONATPOLYMERISIERUNG
PROCÉDÉ DE RÉCUPÉRATION DE PHÉNOL DANS UNE POLYMÉRISATION D'UN POLYCARBONATE EN FUSION

(30) Priority: 06.09.2017 EP 17382596
(43) Date of publication of application: 15.07.2020
(73) Proprietor: SABIC Global Technologies B.V., 4612 PX Bergen op Zoom (NL)
(72) Inventor: IMAM, Rayees Ahamed, Bangalore Karnataka 562125 (IN); GUNALE, Tukaram, Bangalore Karnataka 562125 (IN); ANAPAT, Samir, Bangalore Karnataka 56212 (IN); MATEOS SALVADOR, Fernan, 30390 Cartagena (ES)
(74) Representative: Algemeen Octrooi- en Merkenbureau B.V.
(86) International application number: PCT/IB2018/056749
(87) International publication number: WO 2019/049027

(56) References cited:
- US-B1- 6 277 945

## Description

### CROSS-REFERENCE TO RELATED APPLICATION

This application claims the benefit of European Patent Application Serial No. 17382596 filed September 6, 2017.

### BACKGROUND

During the melt polymerization of bisphenol A (BPA) polycarbonate, the polymerization occurs in polymerization units that operate at high vacuum levels of up to 0.5 millibar in order to remove a reaction vapor comprising the phenol by-product, driving polymerization. The phenol by-product is in the solid phase as the pressure in the polymerization units is below the triple point of the phenol and also due to the presence of diphenyl carbonate (up to 60% weight by weight) that further increases the freezing point of the removed vapor. Therefore, in order to recover the phenol from the removed vapor, the industry today generally employs a minimum of two freeze condensers per polymerization unit that operate alternatively, where one of the condensers collects the solid phase phenol from the reaction vapor and the other condenser melts the collected solid phase phenol by melting at a higher pressure and temperature. US 6,277,945 discloses a method for producing an aromatic polycarbonate comprisisng feeding acetone and a phenol material to a reactor to effect a reaction between said acetone and said phenol material, thereby producing bisphenol A, and polymerizing said bisphenol A with diphenyl carbonate in a polymerizer to produce an aromatic polycarbonate while producing phenol as a by-product, wherein said by-product phenol is recovered from said polymerizer as a crude phenol product containing said by-product phenol as a main component and containing impurities, and said crude phenol product is used as at least a part of said phenol material for producing bisphenol A.

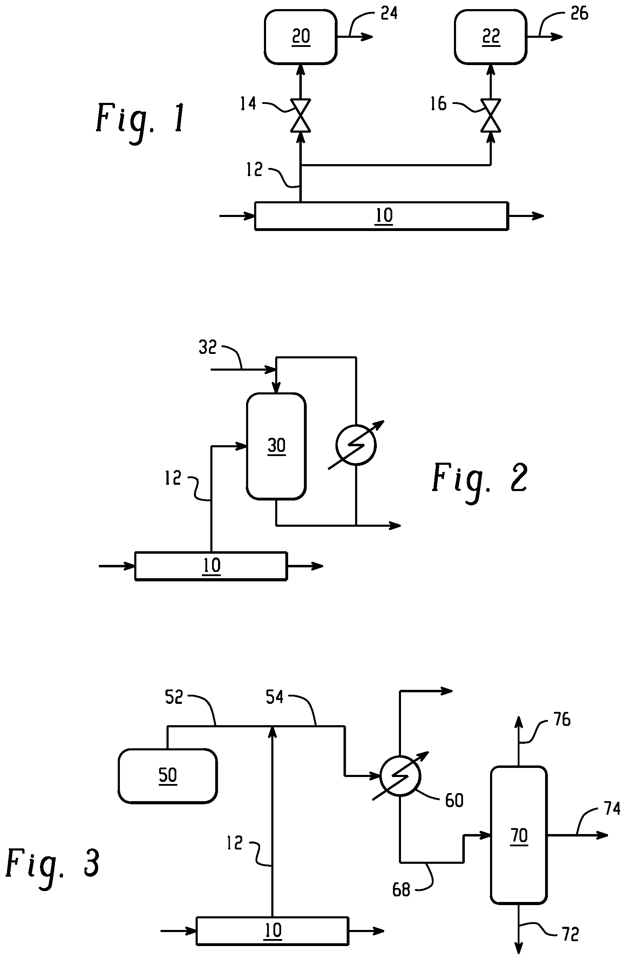

An example of such a system is illustrated in FIG. 1. In FIG. 1, a BPA polycarbonate is polymerized in polymerization unit 10. Overhead stream 12 comprising phenol vapor is removed from polymerization unit 10 and is directed to one of two freeze condensers 20 and 22 via specialty values 14 and 16 to produce freeze condensed streams 24 and 26, respectively. As the utility between the two condensers is constantly being switched from chilled water to steam depending on the operation mode, the design of such condensers that can withstand the thermal stresses caused by switching of utility is extremely challenging. For example, the condensers must be designed to bear the heat load despite the fouling of heat transfer surface by frozen phenol; especially towards the end of an operational cycle. Furthermore, turning the flow of the vapor stream to the condensers on and off depending on the mode of operation is done by large, specially designed valves. These specialized values are designed to operate at high vacuum conditions and large vapor volumetric flows, while still being able to exhibit a very low pressure drop. In summary, fouling, interrupted cyclic operations, and exotic valve design make the overhead processing of melt polymerization units expensive both from an operational stand point as well as from the perspective of capital cost.

Another method for recovering phenol from the overhead streams during a melt polycarbonate polymerization utilizes a liquid scrubbing solution of phenol and a liquid freezing-point depressant to prevent solidification of the recovered phenol from the polymerization units. An example of such a method is illustrated in FIG. 2 that illustrates overhead stream 12 being removed from polymerization unit 10 and directed towards wet scrubber 30. Liquid scrubbing solution 32 is sprayed into a top portion of wet scrubber 30 so that liquid scrubbing solution 32 makes contact with the phenol vapor, condensing the vapor and absorbing it into the liquid scrubbing solution. Even though, from a thermodynamic stand point, it is feasible to use a wet scrubber, from an applicability stand point there are significant challenges to using such a scrubber in industrial settings. For example, the scrubbing solution must be recovered and recycled, increasing both the capital and operating costs. Furthermore, this method of recovering the phenol from the overhead streams requires an extremely low pressure drop of less than 0.5 millibar and such a low pressure drop can lead to difficulty in maintaining the required vacuum levels in the polymerization units.

Attempts at addressing the challenges associated with the low pressure drop have been made. For example, it was found that the polymerization units could be operated at higher pressures by introducing an inert gas such as nitrogen into the polymerization mixture. A gas mixture comprising the phenol by-product and the inert gas is then removed and added to a wet scrubber. This approach though has several limitations as the effective distribution of the inert gas in the polymerization mixture at a large scale in individual, industrial polymerization units is difficult, where a high vapor velocity in the unit results in issues such as foaming, entrainment of oligomers and polymer, and/or clogging of scrubbers and vacuum units. These problems are exacerbated by the presence of the multiple oligomerization and polymerization units present in a polymerization facility that each produce an overhead stream comprising the phenol by-product. Here, each unit produces a polycarbonate with increasing viscosity, and the pressure and velocity parameters of each polymerization unit needs to be adjusted separately to ensure an effective distribution without resulting in foaming. In practice, the economics would be expensive to have a dedicated scrubber for every polymerization unit and a common scrubber for multiple reactors would take away the degree of freedom to control reactor pressures.

U.S. Patent 6,277,945 discloses a method for producing an aromatic polycarbonate, which comprises reacting acetone with a phenol material, thereby producing bisphenol A, and polymerizing the resultant bisphenol A with diphenyl carbonate to produce an aromatic polycarbonate while producing phenol as a by-product, wherein the by-product phenol is recovered as a crude phenol product containing the by-product phenol as a main component and containing impurities, and the crude phenol product is used as at least a part of the phenol material for producing bisphenol A.

Improved methods of recovering phenol from the overhead streams during a melt polycarbonate polymerization are therefore desired.

### BRIEF SUMMARY

Disclosed herein is a method of manufacture of a melt polycarbonate comprising recovering phenol by co-condensation with a solvent in the gas phase.

In an embodiment, a method of manufacture of a melt polycarbonate comprises melt polymerizing a bisphenol and diphenyl carbonate to form phenol and the melt polycarbonate in a polymerization unit; removing an overhead stream comprising the phenol in a gas phase from the polymerization unit; combining the overhead stream with a gas stream comprising a solvent in a gas phase to form a combined gas stream; wherein the combined gas stream has a freezing point at less than 3 mbar absolute that is less than the triple point of phenol; and condensing the combined gas stream in a condenser to form a condensed stream comprising the phenol and the solvent in a liquid phase.

The above described and other features are exemplified by the following figures, detailed description, and claims.

### BRIEF DESCRIPTION OF THE DRAWINGS

The following Figures are exemplary embodiments, which are provided to illustrate the present method, wherein the like elements are numbered alike. The figures are illustrative and are not intended to limit devices made in accordance with the disclosure to the materials, conditions, or process parameters set forth herein.
FIG. 1 is an illustration of an embodiment of a prior method of recovering phenol from an overhead stream using freeze condensers;
FIG. 2 is an illustration of an embodiment of a prior method of recovering phenol from an overhead stream using a liquid solvent;
FIG. 3 is an illustration of an embodiment of a method of recovering phenol from an overhead stream using a gas phase solvent;
FIG. 4 is an illustration of an embodiment of a separation column with a side draw;
FIG. 5 is an illustration of an embodiment of a separation column with a side draw and a flash unit;
FIG. 6 is an illustration of an embodiment of a separation column in fluid communication with a downstream separation column; and
FIG. 7 is an illustration of an embodiment of a separation column in fluid communication with a downstream separation column and a flash unit.

### DETAILED DESCRIPTION

A novel method to recover phenol from the overhead streams of a melt polymerization was developed by co-condensation of the phenol and a gas phase solvent. The method comprises melt polymerizing a bisphenol and diphenyl carbonate to form phenol and a polycarbonate in a polymerization unit; removing an overhead stream comprising the phenol in a gas phase from the polymerization unit; combining the overhead stream with a gas stream to form a combined gas stream; wherein the combined gas stream has a freezing point at lower than 3 millibar absolute (mbar) that is less than the triple point of phenol of 314 Kelvin. The present method is a non-scrubber based solution that has several advantages over freeze condensation methods including the avoidance of the cyclic operation of the freeze condensers, which enables the continuous operation of the condenser; a potential reduction in the number of condensers required when compared to the conventional process that is characterized by multiple freeze condensers per reactor; an increase in reliability of the condenser as repeated thermal cycling of the current condenser is avoided; and a simplified plant layout that can avoid the use of specialized butterfly values.

The "polycarbonate" as used herein is derived from diphenyl carbonate and a bisphenol and can have repeating structural carbonate units of formula (1) 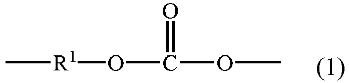 in which the R¹ groups contain aliphatic, alicyclic, and/or aromatic moieties (e.g., greater than or equal to 30 percent, or greater than or equal to 60 percent of the total number of R¹ groups can contain aromatic moieties and the balance thereof are aliphatic or alicyclic). Optionally, each R¹ can be a C₆₋₃₀ aromatic group that can contain at least one aromatic moiety. R¹ can be derived from the bisphenol.

The bisphenol can comprise a bisphenol of the formula HO-R¹-OH, wherein the R¹ group can contain an aliphatic, an alicyclic, or an aromatic moiety. For example, the bisphenol can have the formula (2)

HO-A¹-Y¹-A²-OH (2)

wherein each of A¹ and A² is a monocyclic divalent aromatic group and Y¹ is a single bond or a bridging group having one or more atoms that separate A¹ from A². One atom can separate A¹ from A².

The bisphenol can have the formula (3) 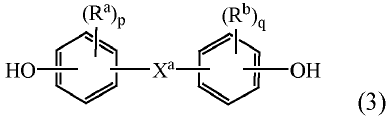 wherein R^{a} and R^{b} are each independently a halogen, C₁₋₁₂ alkoxy, or C₁₋₁₂ alkyl; and p and q are each independently integers of 0 to 4. It will be understood that R^{a} is hydrogen when p is 0, and likewise R^{b} is hydrogen when q is 0. Also in formula (3), X^{a} is a bridging group connecting the two hydroxy-substituted aromatic groups, where the bridging group and the hydroxy substituent of each C₆ arylene group are disposed ortho, meta, or para (specifically, para) to each other on the C₆ arylene group. The bridging group X^{a} can be single bond, -O-, -S-, -S(O)-, -S(O)₂-, -C(O)-, or a C₁₋₁₈ organic bridging group. The C₁₋₁₈ organic bridging group can be cyclic or acyclic, aromatic or non-aromatic, and can further comprise heteroatoms, for example, halogens, oxygen, nitrogen, sulfur, silicon, or phosphorous. The C₁₋₁₈ organic bridging group can be disposed such that the C₆ arylene groups connected thereto are each connected to a common alkylidene carbon or to different carbons of the C₁₋₁₈ organic bridging group. In formula (3), p and q can each be 1, and R^{a} and R^{b} are each a C₁₋₃ alkyl group, specifically, methyl, disposed meta to the hydroxy group on each arylene group.

X^{a} can be a substituted or unsubstituted C₃₋₁₈ cycloalkylidene, a C₁₋₂₅ alkylidene of formula -C(R^{c})(R^{d})- wherein R^{c} and R^{d} are each independently hydrogen, C₁₋₁₂ alkyl, C₁₋₁₂ cycloalkyl, C₇₋₁₂ arylalkyl, C₁₋₁₂ heteroalkyl, or cyclic C₇₋₁₂ heteroarylalkyl, or a group of the formula -C(=R^{e})- wherein R^{e} is a divalent C₁₋₁₂ hydrocarbon group. Groups of this type include methylene, cyclohexylmethylene, ethylidene, neopentylidene, or isopropylidene, as well as 2-[2.2.1]-bicycloheptylidene, cyclohexylidene, cyclopentylidene, cyclododecylidene, or adamantylidene.

X^{a} can be a C₁₋₁₈ alkylene group, a C₃₋₁₈ cycloalkylene group, a fused C₆₋₁₈ cycloalkylene group, or a group of the formula -B¹-G-B²- wherein B¹ and B² are the same or different C₁₋₆ alkylene group and G is a C₃₋₁₂ cycloalkylidene group or a C₆₋₁₆ arylene group. For example, X^{a} can be a substituted C₃₋₁₈ cycloalkylidene of formula (4) 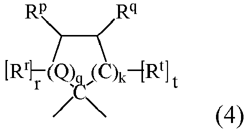 wherein R^{r}, R^{p}, R^{q}, and R^{t} are each independently hydrogen, halogen, oxygen, or C₁₋₁₂ hydrocarbon groups; Q is a direct bond, a carbon, or a divalent oxygen, sulfur, or -N(Z)-where Z is hydrogen, halogen, hydroxy, C₁₋₁₂ alkyl, C₁₋₁₂ alkoxy, or C₁₋₁₂ acyl; r is 0 to 2, t is 1 or 2, q is 0 or 1, and k is 0 to 3, with the proviso that at least two of R^{r}, R^{p}, R^{q}, and R^{t} taken together are a fused cycloaliphatic, aromatic, or heteroaromatic ring. It will be understood that where the fused ring is aromatic, the ring as shown in formula (4) will have an unsaturated carbon-carbon linkage where the ring is fused. When k is one and q is 0, the ring as shown in formula (4) contains 4 carbon atoms, when k is 2, the ring as shown in formula (4) contains 5 carbon atoms, and when k is 3, the ring contains 6 carbon atoms. Two adjacent groups (e.g., R^{q} and R^{t} taken together) can form an aromatic group or R^{q} and R^{t} taken together can form one aromatic group and R^{r} and R^{p} taken together form a second aromatic group. When R^{q} and R^{t} taken together form an aromatic group, R^{p} can be a double-bonded oxygen atom, i.e., a ketone.

Specific examples of bisphenol compounds of formula (3) include 1,1-bis(4-hydroxyphenyl) methane, 1,1-bis(4-hydroxyphenyl) ethane, 2,2-bis(4-hydroxyphenyl) propane (hereinafter "bisphenol A" or "BPA"), 2,2-bis(4-hydroxyphenyl) butane, 2,2-bis(4-hydroxyphenyl) octane, 1,1-bis(4-hydroxyphenyl) propane, 1,1-bis(4-hydroxyphenyl) n-butane, 2,2-bis(4-hydroxy-2-methylphenyl) propane, 1,1-bis(4-hydroxy-t-butylphenyl) propane, 3,3-bis(4-hydroxyphenyl) phthalimidine, 2-phenyl-3,3-bis(4-hydroxyphenyl) phthalimidine (PPPBP), or 1,1-bis(4-hydroxy-3-methylphenyl)cyclohexane (DMBPC). Combinations comprising at least one of the foregoing bisphenols can also be used. The bisphenol can comprise bisphenol A, in which each of A¹ and A² can be p-phenylene, and Y¹ can be isopropylidene in formula (3).

A catalyst can be used to facilitate the polycarbonate polymerization. The catalyst can comprise at least one of a quaternary catalyst or an alkali catalyst. The quaternary catalyst comprises at least one of a quaternary ammonium compound or a quaternary phosphonium compound. The quaternary ammonium compound can be a compound of the structure (R⁴)₄N⁺X⁻, wherein each R⁴ is the same or different, and is a C₁₋₂₀ alkyl, a C₄₋₂₀ cycloalkyl, or a C₄₋₂₀ aryl; and X⁻ is an organic or inorganic anion, for example, a hydroxide, halide, carboxylate, sulfonate, sulfate, formate, carbonate, or bicarbonate. Examples of organic quaternary ammonium compounds include tetramethyl ammonium hydroxide, tetrabutyl ammonium hydroxide, tetramethyl ammonium acetate, tetramethyl ammonium formate, or tetrabutyl ammonium acetate.

The quaternary phosphonium compound can be a compound of the structure (R⁵)₄P⁺X⁻, wherein each R⁵ is the same or different, and is a C₁₋₂₀ alkyl, a C₄₋₂₀ cycloalkyl, or a C₄₋₂₀ aryl; and X⁻ is an organic or inorganic anion, for example, a hydroxide, phenoxide, halide, carboxylate, for example, acetate or formate, sulfonate, sulfate, formate, carbonate, or bicarbonate. Where X⁻ is a polyvalent anion, for example, carbonate or sulfate, it is understood that the positive and negative charges in the quaternary ammonium and phosphonium structures are properly balanced. For example, where each R⁵ are methyl and X⁻ is carbonate, it is understood that X⁻ represents 2(CO₃⁻²).

Examples of organic quaternary phosphonium compounds include tetramethyl phosphonium hydroxide, tetramethyl phosphonium acetate, tetramethyl phosphonium formate, tetrabutyl phosphonium hydroxide, tetraethyl phosphonium acetate, tetrapropyl phosphonium acetate, tetrabutyl phosphonium acetate (TBPA) , tetrapentyl phosphonium acetate, tetrahexyl phosphonium acetate, tetraheptyl phosphonium acetate, tetraoctyl phosphonium acetate, tetradecyl phosphonium acetate, tetradodecyl phosphonium acetate, tetratolyl phosphonium acetate, tetramethyl phosphonium benzoate, tetraethyl phosphonium benzoate, tetrapropyl phosphonium benzoate, tetraphenyl phosphonium benzoate, tetraethyl phosphonium formate, tetrapropyl phosphonium formate, tetraphenyl phosphonium formate, tetramethyl phosphonium propionate, tetraethyl phosphonium propionate, tetrapropyl phosphonium propionate, tetramethyl phosphonium butyrate, tetraethyl phosphonium butyrate, tetrapropyl phosphonium butyrate, tetraphenyl phosphonium acetate (TPPA), or tetraphenyl phosphonium phenoxide (TPPP). The quaternary catalyst can comprise at least one of tetrabutyl phosphonium acetate, TPPP, or TPPA.

The amount of the quaternary catalyst can be added based upon the total number of moles of bisphenol employed in the polymerization reaction. When referring to the ratio of catalyst, for example, phosphonium salt, to all bisphenols employed in the polymerization reaction, it is convenient to refer to moles of phosphonium salt per mole of the bisphenol(s), meaning the number of moles of phosphonium salt divided by the sum of the moles of each individual bisphenol present in the reaction mixture. The amount of the optional quaternary catalyst (e.g., organic ammonium or phosphonium salts) can each independently be employed in an amount of 1 × 10⁻² to 1 × 10⁻⁵, or 1 × 10⁻³ to 1 × 10⁻⁴ moles per total mole of the bisphenol(s) in the monomer mixture.

The alkali catalyst comprises a source of one or both of alkali ions or alkaline earth ions. The sources of these ions can include alkaline earth hydroxides, for example, magnesium hydroxide or calcium hydroxide. Sources of alkali metal ions can include the alkali metal hydroxides, for example, at least one of lithium hydroxide, sodium hydroxide, or potassium hydroxide. Examples of alkaline earth metal hydroxides are calcium hydroxide or magnesium hydroxide. The alkali catalyst can comprise sodium hydroxide. Other possible sources of alkaline earth or alkali metal ions include salts of carboxylic acids (for example, sodium acetate) or derivatives of ethylene diamine tetraacetic acid (EDTA) (for example, EDTA tetrasodium salt or EDTA magnesium disodium salt). For example, the alkali catalyst can comprise at least one of an alkali metal salt(s) of a carboxylic acid or an alkaline earth metal salt(s) of a carboxylic acid. In another example, the alkali catalyst comprises Na₂Mg EDTA or a salt thereof.

The alkali catalyst can also, or alternatively, comprise salt(s) of a non-volatile inorganic acid. For example, the alkali catalyst can comprise at least one of NaH₂PO₃, NaH₂PO₄, Na₂HPO₃, KH₂PO₄, CsH₂PO₄, or Cs₂HPO₄. Alternatively, or in addition, the alkali catalyst can comprise mixed alkali metal salt(s) of phosphoric acid, for example, at least one of NaKHPO₄, CsNaHPO₄, or CsKHPO₄. The alkali catalyst can comprise KNaHPO₄, wherein a molar ratio of Na to K is 0.5 to 2.

The alkali catalyst typically can be used in an amount sufficient to provide 1 × 10⁻² to 1 × 10⁻⁸ moles, or 1 × 10⁻⁴ to 1 × 10⁻⁷ moles of metal hydroxide per mole of the bisphenol(s).

Quenching of the transesterification catalysts and any reactive catalysts residues with an acidic compound after polymerization can be completed and can be useful in some melt polymerization processes. Among the many quenchers that can be used are alkyl sulfonic esters of the formula R⁸SO₃R⁹ wherein R⁸ is hydrogen, C₁₋₁₂ alkyl, C₆₋₁₈ aryl, or C₇₋₁₉ alkylaryl, and R⁹ is C₁₋₁₂ alkyl, C₆₋₁₈ aryl, or C₇₋₁₉ alkylaryl. Examples of quenchers include benzenesulfonate, p-toluenesulfonate, methylbenzene sulfonate, ethylbenzene sulfonate, n-butyl benzenesulfonate, octyl benzenesulfonate, phenyl benzenesulfonate, methyl p-toluenesulfonate, ethyl p-toluenesulfonate, n-butyl p-toluene sulfonate, octyl p-toluenesulfonate, or phenyl p-toluenesulfonate. In particular, the quencher can comprise an alkyl tosylate, for example, n-butyl tosylate.

In the melt polymerization method, the polycarbonate can be prepared by reacting, in a molten state, the diphenyl carbonate and the bisphenol in the presence of the catalyst. The reaction can be carried out in typical polymerization equipment, such as a continuously stirred reactor (CSTR), plug flow reactor, wire wetting fall polymerizers, free fall polymerizers, horizontal polymerizers, wiped film polymerizers, BANBURY mixers, single or twin screw extruders, or a combination comprising one or more of the foregoing. Volatile by-products, such as phenol, are removed from the molten reactants and the polymer is isolated as a molten residue. Melt polymerization can be conducted as a batch process or as a continuous process. In either case, the melt polymerization conditions used can comprise two or more distinct reaction stages.

For example, the polymerization can comprise an oligomerization stage in which the starting bisphenol and diphenyl carbonate are converted into an oligomeric polycarbonate and a second reaction stage also referred to as a polymerization stage wherein the oligomeric polycarbonate formed in the oligomerization stage is converted to high molecular weight polycarbonate. The reaction stages can be performed in reaction vessels under low (for example, increasingly low) pressures to remove the phenol, shifting the reaction towards polymerization. The oligomerization stage can comprise 1 or more, or 2 or more, or 2 to 4 oligomerization units (for example, 2 to 4 continuously stirred tanks). When 2 or more oligomerization units are present in series, one or both of an increase in temperature or a decrease in pressure can occur from one unit to the next.

The oligomerization stage can comprise a first oligomerization unit located in series and upstream of a second oligomerization unit. The temperature in a first oligomerization unit can be 160 to 300 degrees Celsius (°C), or 160 to 275°C, or 160 to 250°C, or 200 to 270°C, or 230 to 270°C. The pressure in a first oligomerization unit can be 50 to 200 mbar, or 75 to 200 mbar. The viscosity of the stream exiting the first oligomerization unit can be 0.05 to 1 Pascal seconds (Pa s), or 0.05 to 0.5 Pa s. The temperature in a second oligomerization unit can be 250 to 300°C, or 270 to 300°C. The pressure in a second oligomerization unit can be 5 to 50 mbar, or 10 to 40 mbar. The viscosity of the stream exiting the second oligomerization unit can be 0.5 to 10 Pa s, or 1 to 5 Pa s, or greater than or equal to 1 Pa s.

The polymerization stage can comprise 1 or more, or 2 or more, or 2 polymerization units (for example, wire wetting fall polymerization units, horizontal polymerizers, vertical polymerizers, reactive extruders, or a continuously stirred tanks) located downstream of the oligomerization units. The polymerization stage can occur at a temperature of 240 to 350°C, or 280 to 300°C, or 240 to 270°C, or 250 to 310°C. The polymerization can occur in a series of polymerization units that can each individually have increasing temperature and/or vacuum. The polymerization stage can comprise a first polymerization unit located in series and upstream of a second polymerization unit. The first polymerization unit can be at a temperature of 240 to 350°C, or 260 to 310°C and a pressure of 100 to 1,100 mbar, or 250 to 900 mbar. The second polymerization unit can be at a temperature of 240 to 350°C, or 260 to 300°C and a pressure of less than or equal to 600 mbar, or 100 to 500 mbar.

An overhead stream from one or more of an oligomerization unit and a polymerization unit can be combined with a gas stream comprising a solvent in a gas phase to form a combined gas stream that has a freezing point at less than 3 mbar that is less than the triple point of phenol. For example, the freezing point of the combined gas stream at less than 3 mbar can be less than or equal to 40°C, or less than or equal to 35°C, or 0 to 35°C. The combining can comprise combining the two streams into one stream via a Y-joint or a T-joint. The combining can comprise passing the combined stream through a static mixer or an ejector type mixer. The combined gas stream can comprise 30 to 90 weight percent (wt%), or 30 to 70 wt% of the overhead stream based on the total weight of the combined gas stream. The gas stream, prior to combining with the overhead stream, can comprise 90 to 100 vol%, or 95 to less than 100 vol% of the solvent based on the total weight of the gas stream. The combined gas stream can comprise 10 to 70 wt%, or 30 to 70 wt% of the solvent based on the total weight of the combined gas stream.

The solvent can have a freezing point less than that of phenol, for example, of less than or equal to 40°C, or less than or equal to 30°C, or -15 to 30°C, or -15 to 0°C at 1 atmosphere pressure. The solvent can have a volatility of less than that of phenol, for example, the solvent can have a boiling point of greater than that of phenol, or greater than or equal to 180°C, or 190 to 250°C at 1 atmosphere pressure. The solvent can have a vapor pressure at 15°C of less than or equal to 0.5 mbar, or less than or equal to 0.4 mbar, or 0.01 to 0.5 mbar.

The solvent can comprise at least one of an aryl alkyl carbonate, a dialkyl carbonate, or an alkylene glycol. The solvent can comprise at least one of phenyl methyl carbonate, anisole, ethylene glycol, or propylene glycol. The solvent can comprise a compound present in the melt polymerization such as at least one of anisole or phenyl methyl carbonate.

The combined gas stream can be condensed in a condenser to form a condensed stream comprising the phenol and the solvent in a liquid phase. The only phases present in the condenser of the phenol and the solvent can be the liquid and vapor phases. Examples of condensers include knock out drums and spiral condensers. A condenser having a shell and tube design having a low pressure drop, such as those having a cross flow, can be utilized.

FIG. 3 is an illustration of an embodiment of a method of recovering phenol from an overhead stream using a solvent in the gas phase. FIG. 3 shows overhead stream 12 from polymerization unit 10 being combined with gas stream 52 to form combined gas stream 54. Gas stream 52 can originate from evaporator 50 that heats the liquid solvent to a temperature greater than the boiling point of the solvent to form the gas phase solvent. Combined gas stream 54 can be directed to condenser 60 to form condensed stream 68 comprising the phenol from overhead stream 12 and the solvent in the liquid phase.

The condensed stream can then be separated into two or more streams to recover the phenol and the solvent. For example, the condensed stream can be separated in a separation column to form at least three streams including a diphenyl carbonate recovery stream comprising diphenyl carbonate, a solvent recovery stream comprising the solvent, and a phenol recovery stream comprising phenol. The diphenyl carbonate recovery stream can comprise 80 to 95 wt%, or 85 to 95 wt% of diphenyl carbonate based on the total weight of the diphenyl carbonate recovery stream. The diphenyl carbonate recovery stream can comprise 0 to 1 wt% of each of phenol and the solvent independently based on the total weight of the diphenyl carbonate recovery stream. The solvent recovery stream can comprise 80 to 99 wt%, or 85 to 95 wt% of the solvent based on the total weight of the solvent recovery stream. The phenol recovery stream can comprise 95 to 100 wt%, or 99 to 100 wt% of phenol based on the total weight of the phenol recovery stream.

FIG. 3 illustrates that condensed stream 68 can be directed to separation column 70 and separated into diphenyl carbonate recovery stream 72, solvent recovery stream 74, and a phenol recovery stream 76. Diphenyl carbonate recovery stream 72 can be removed from separation column 70 as a bottom stream from a bottom side of separation column 70. Solvent recovery stream 74 can be removed from separation column 70 as a side draw. Phenol recovery stream 76 can be removed from separation column 70 as a top stream from a top side of separation column 70.

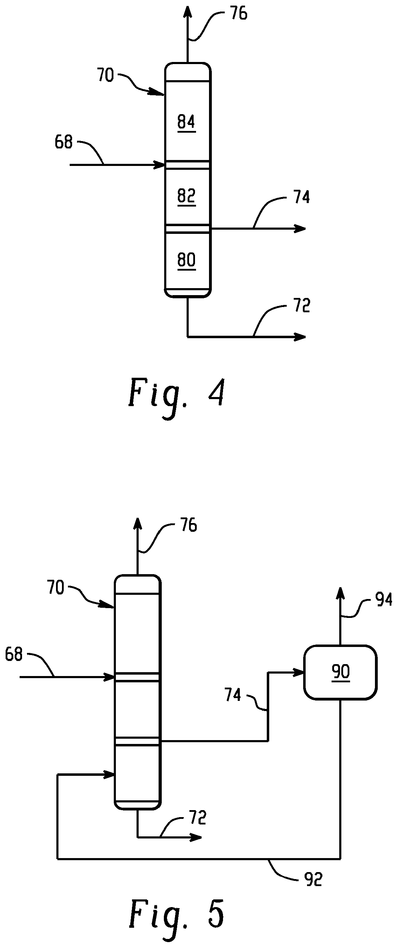

FIG. 4 illustrates a specific example of such a separation. Here, separation column 70 is separated into three different packing sections. Condensed stream 68 is directed to separation column 70 in between middle packing 82 and upper packing section 84. Diphenyl carbonate recovery stream 72 is removed from separation column 70 as a bottom stream from a bottom side of separation column 70. Phenol recovery stream 76 is removed from separation column 70 as a top stream from a top side of separation column 70. Solvent recovery stream 74 can be removed from separation column 70 as a vapor side draw or a liquid side draw at a location in between lower packing section 80 and middle packing section 82. Removing the solvent recovery stream 74 as a vapor side draw can be beneficial as the solvent would not need to be re-vaporized if it was to be recycled for mixing with the overhead stream.

When the solvent recovery stream 74 is removed as a liquid side draw, the solvent recovery stream can be further separated, for example, in a flash unit to further purify the solvent and to re-vaporize it so that it could be recycled for mixing with the overhead stream. FIG. 5 illustrates such a separation, where solvent recovery stream 74 is directed to flash unit 90 to form recycle stream 92 and flashed solvent vapor stream 94. Recycle stream 92 can be directed back to separation column 70 and can be added, for example, to lower packing section 80. Recycle stream 92 can comprise 20 to 60 wt%, or 30 to 50 wt% of diphenyl carbonate based on a total weight of the recycle stream. Recycle stream 92 can comprise 30 to 70 wt%, or 40 to 60 wt% of solvent based on a total weight of the recycle stream. Flashed solvent vapor stream 94 can comprise 70 to 99 wt%, or 80 to 100 wt%, or 90 to 99 wt% of solvent based on a total weight of the flashed solvent vapor stream.

Alternatively, the condensed stream can be first separated in a separation column to form at least two streams including a bottom stream and a phenol recovery stream, where the bottom stream can then be separated in a downstream separation column into a solvent recovery stream comprising the solvent. The solvent recovery stream can comprise 1 to 20 wt%, or 5 to 15 wt%, or 10 to 15 wt% of the solvent based on the total weight of the solvent recovery stream.

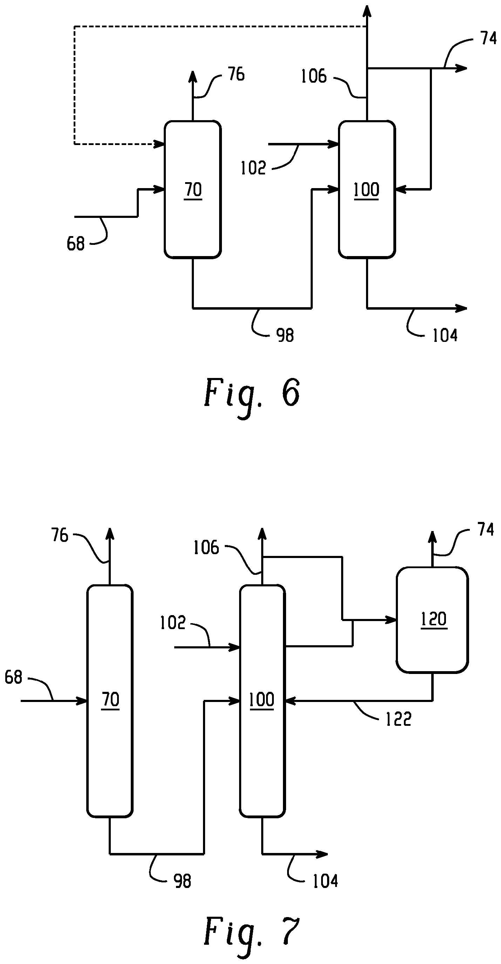

For example, FIG. 6 and FIG. 7 illustrate that condensed stream 68 can be separated in the separation column 70 into bottom stream 98 and phenol recovery stream 76 and that bottom stream 98 can be directed to a downstream separation process for further separation. Bottom stream 98 can comprise 60 to 95 wt%, or 80 to 95 wt% diphenyl carbonate and 1 to 20 wt%, or 5 to 15 wt%, or 10 to 15 wt% of solvent based on the total weight of the bottom stream. Side feed stream 102 can be added to downstream separation column 100. Downstream separation column 100 can be a separation column from a diphenyl carbonate production facility that separates phenyl methyl carbonate and diphenyl carbonate from side feed stream 102. Side feed stream 102 can comprise diphenyl carbonate or phenyl methyl carbonate. Solvent recovery stream 74 can comprise 70 to 99 wt%, or 80 to 99 wt%, or 85 to 95 wt% of the solvent based on the total weight of the solvent recovery stream. Heavies stream 104 can comprise 90 to 100 wt%, or 95 to 100 wt%, or 90 to 95 wt% of diphenyl carbonate based on the total weight of the heavies stream.

FIG. 6 illustrates that downstream separation column 100 can separate bottom stream 98 into top stream 106 and heavies stream 104. At least a portion of top stream 106 can be separated out as solvent recovery stream 74 that can be directed to a downstream process. At least a portion of top stream 106 can be directed back to separation column 70. At least a portion of top stream 106 can be directed back to downstream separation column 100. FIG. 6 has the benefit of utilizing existing equipment in melt polymerization facilities.

FIG. 7 illustrates that downstream separation column 100 can separate bottom stream 98 into top stream 106 and heavies stream 104. At least a portion of top stream 106 can be directed to flash unit 120 to form solvent recovery stream 74 and return stream 122. Return stream 122 can be directed back into downstream separation column 100. A liquid side draw can also be removed from downstream separation column 100 and directed to flash unit 120. At least a portion of one or both of solvent recovery stream 74 or top stream 106 can be directed back to downstream separation column 100. At least a portion of one or both of solvent recovery stream 74 or top stream 106 can be directed back to separation column 70. At least a portion of one or both of solvent recovery stream 74 or top stream 106 can be removed from the separation and can be directed to a downstream process. As FIG. 7 utilizes a liquid side draw to remove a portion of the solvent, a higher purity of the solvent in the solvent recovery stream as compared to the separation as illustrated in FIG. 6 can be achieved, for example, of greater than 93 wt%.

One or more of the recovered streams can be in fluid communication with a monomer production facility such as at least one of a diphenyl carbonate production facility or a bisphenol A production facility. For example, at least a portion of the phenol recovery stream, such as phenol recovery stream 76, can be directed to a diphenyl carbonate production facility, where the phenol can be reacted using a phosgenation reaction, a carbonylation reaction, or a transesterification method to produce diphenyl carbonate. Likewise, at least a portion of the phenol recovery stream can be directed to a bisphenol A carbonate production facility, where the phenol can be reacted with acetone process to produce bisphenol A. When the solvent comprises phenyl methyl carbonate, at least a portion of a solvent recovery stream such as solvent recovery stream 74 or flashed solvent recovery stream 94 can be directed to a diphenyl carbonate production facility.

One or more of the recovered streams can be in direct fluid communication with a monomer production facility for immediate use. Conversely, a recovered stream can be in fluid communication with a storage unit, the recovered stream can be stored for an amount of time, optionally transported, and then the stored recovered stream can be directed to a monomer production facility.

The following examples are provided to illustrate the present method. The examples are merely illustrative and are not intended to limit devices made in accordance with the disclosure to the materials, conditions, or process parameters set forth therein.

### Examples

### Examples 1-2: Continuous condensation of a phenol solvent mixture

A lab scale distillation column was set up where a single-neck round-bottom flask was heated using an oil bath. A jacketed distillation column was attached to the neck of the round-bottom flask and cooling water was flowed in the jacket of the distillation entering from a lower inlet and exiting from an upper outlet. A vacuum pump was hooked up to the top of the distillation column to control the pressure in the column.

In Example 1, a solution comprising 66.6 volume percent (vol%) of phenol and 33.4 vol% of diphenyl carbonate was added to the round-bottom flask. A temperature of the entering cooling water was 30°C and the pressure was set to 1.5 millibar. Severe freezing was observed in the distillation column as soon as it started boiling.

In Example 2, a solution comprising 30.1 vol% of phenol, 15.1 vol% of diphenyl carbonate, and 54.8 vol% phenyl methyl carbonate was added to the round-bottom flask. A temperature of the entering cooling water was 5°C and the pressure was set to 0.6 millibar. The experiment was continuously run in total reflux mode for several days and no freezing was observed. This result is particularly surprising as compared to Example 1 as both the temperature and the pressure are significantly lower and still no freezing was observed.

### Example 3-5: Effect of solvent presence in the separation column

In Example 3, a simulation using ASPEN software was performed using separation column 70 as illustrated in FIG. 6 using a feed location in between the middle packing section and the lower packing section and instead using the feed stream that was freeze condensed stream 24 as illustrated in FIG. 1. The results are shown in Table 1, where the total mass flow rate is shown in kilograms per hour (kg/hr).

| Table 1 | | | |
|---|---|---|---|
| Stream | Feed stream | Bottom stream | Phenol recovery stream |
| Stream number | 24 | 98 | 76 |
| Total mass flow rate (kg/hr) | 2,500 | 800 | 1,700 |
| Phenol (wt%) | 69 | 5 | 100 |
| Diphenyl carbonate (wt%) | 28 | 88 | - |
| Bisphenol A (wt%) | 2 | 5 | - |
| Other (wt%) | 1 | 2 | - |
| Pressure (bar absolute) | 1 | 0.15 | 0.07 |
| Temperature (°C) | 135 | 190 | 107 |

In Example 4, a simulation using ASPEN software was performed using separation column 70 as illustrated in FIG. 4. The results are shown in Table 2.

| Table 2 | | | | |
|---|---|---|---|---|
| Stream | Feed stream | Diphenyl carbonate recovery stream | Solvent recovery stream | Phenol recovery stream |
| Stream number | 68 | 72 | 74 | 76 |
| Total mass flow rate (kg/hr) | 3,031 | 772 | 530 | 1728 |
| Phenol (wt%) | 58 | - | 5 | 100 |
| Diphenyl carbonate (wt%) | 23 | 91 | - | - |
| Phenyl methyl carbonate (wt%) | 17 | - | 94 | - |
| Bisphenol A (wt%) | 1 | 5 | - | - |
| Other (wt%) | 1 | 4 | - | - |
| Pressure (bar absolute) | 1 | 0.15 | 0.12 | 0.07 |
| Temperature (°C) | 135 | 190 | 120 | 107 |

In Example 5, a simulation using ASPEN software was performed in accordance with Example 4 except that the solvent recovery stream 74 was directed towards flash unit 90 as illustrated in FIG. 5. The composition of streams 68, 72, and 76 are the same as shown in Table 2 and the compositions of the remaining streams 74, 92, and 94 are shown in Table 3.

| Table 3 | | | |
|---|---|---|---|
| Stream | Solvent recovery stream | Recycle stream | Flashed solvent vapor stream |
| Stream number | 74 | 92 | 94 |
| Total mass flow rate (kg/hr) | 4,000 | 3,469 | 533 |
| Phenol (wt%) | 2 | 2 | 5 |
| Diphenyl carbonate (wt%) | 36 | 41 | - |
| Phenyl methyl carbonate (wt%) | 61 | 56 | 95 |
| Bisphenol A (wt%) | - | - | - |
| Other (wt%) | 1 | 1 | - |
| Pressure (bar absolute) | 1 | 1 | 1 |
| Temperature (°C) | 135 | 230 | 230 |

Tables 2 and 3 show that the phenyl methyl carbonate solvent can be recovered using a side draw from Column 70.

The compositions, methods, and articles can alternatively comprise, consist of, or consist essentially of, any appropriate materials, steps, or components herein disclosed. The compositions, methods, and articles can additionally, or alternatively, be formulated so as to be devoid, or substantially free, of any materials (or species), steps, or components, that are otherwise not necessary to the achievement of the function or objectives of the compositions, methods, and articles.

The terms "a" and "an" do not denote a limitation of quantity, but rather denote the presence of at least one of the referenced item. The term "or" means "and/or" unless clearly indicated otherwise by context. Reference throughout the specification to "an aspect", "an embodiment", "another embodiment", "some embodiments", and so forth, means that a particular element (e.g., feature, structure, step, or characteristic) described in connection with the embodiment is included in at least one embodiment described herein, and may or may not be present in other embodiments. In addition, it is to be understood that the described elements may be combined in any suitable manner in the various embodiments.

"Optional" or "optionally" means that the subsequently described event or circumstance may or may not occur, and that the description includes instances where the event occurs and instances where it does not. The suffix "(s)" as used herein is intended to include both the singular and the plural of the term that it modifies, thereby including one or more of that term (e.g., the colorant(s) includes one or more colorants).

Unless specified to the contrary herein, all test standards are the most recent standard in effect as of the filing date of this application, or, if priority is claimed, the filing date of the earliest priority application in which the test standard appears.

Unless otherwise mentioned, all weight percent values of a component in a stream are based on the total weight of the respective stream.

The term "combination" is inclusive of blends, mixtures, alloys, reaction products, and the like. Also, "combinations comprising at least one of the foregoing" or "at least one of" means that the list is inclusive of each element individually, as well as combinations of two or more elements of the list, and combinations of at least one element of the list with like elements not named.

Unless defined otherwise, technical and scientific terms used herein have the same meaning as is commonly understood by one of skill in the art to which this disclosure belongs.

## Claims

1. A method of manufacture of a melt polycarbonate, comprising:
melt polymerizing a bisphenol and diphenyl carbonate to form phenol and the melt polycarbonate in a polymerization unit;
removing an overhead stream comprising the phenol in a gas phase from the polymerization unit;
combining the overhead stream with a gas stream comprising a solvent in a gas phase to form a combined gas stream; wherein the combined gas stream has a freezing point at less than 3 mbar absolute that is less than the triple point of phenol; and
condensing the combined gas stream in a condenser to form a condensed stream comprising the phenol and the solvent in a liquid phase.

2. The method of Claim 1, wherein the solvent has at least one of of a freezing point of less than or equal to 40°C, or less than or equal to 35°C at 3 mbar absolute or a vapor pressure at 15°C of less than or equal to 0.5 mbar.

3. The method of any one of the preceding claims, wherein the solvent comprises at least one of an aryl alkyl carbonate, a dialkyl carbonate, anisole, or an alkylene glycol.

4. The method of any one of the preceding claims, wherein the solvent comprises at least one of phenyl methyl carbonate, anisole, ethylene glycol, or propylene glycol.

5. The method of any one of the preceding claims, wherein the combined gas stream comprises 30 to 90 wt%, preferably, 30 to 70 wt% of the overhead stream based on the total weight of the combined stream.

6. The method of any one of the preceding claims, wherein the only phases of the solvent and the phenol in the condenser are liquid and vapor phases.

7. The method of any one of the preceding claims, further comprising
separating the condensed stream in a separation column into a diphenyl carbonate recovery stream, a solvent recovery stream, and a phenol recovery stream; or
separating the condensed stream in the separation column into a bottom stream and the phenol recovery stream, and further separating the bottom stream in a downstream separation column and recovering the solvent recovery stream from the downstream separation column.

8. The method of Claim 7, wherein the separation column comprises an upper section, a middle section, and a lower section; and the method comprises adding the condensed stream in between the middle section and the upper section.

9. The method of any one of Claims 7 to 8, wherein the separating comprises the separating the diphenyl carbonate recovery stream from a bottom end of the separation column, the solvent recovery stream from a side of the separation column, and the phenol recovery stream from a top end of the separation column.

10. The method of Claim 9, further comprising directing the solvent recovery stream to a flash unit; withdrawing a flashed vapor stream and a recycle stream from the flash unit; and directing the recycle stream back into the separation column; wherein the flashed vapor stream comprises 70 to 99 wt% of the solvent based on the total weight of the flashed vapor stream.

11. The method of Claim 7, wherein the process comprises separating the bottom stream from a bottom end of the separation column and the phenol recovery stream from a top end of the separation column; and further separating the bottom stream in the downstream separation column and recovering the solvent recovery stream from the downstream separation column.

12. The method of Claim 11, wherein the recovering the solvent recovery stream from the downstream separation column comprises withdrawing a top stream from the downstream separation column; directing at least a portion of the top stream to a flash unit; withdrawing the solvent recovery stream and a return stream from the flash unit; and directing the return stream to the downstream separation column.

13. The method of any one of Claims 7, 11, or 12, wherein the downstream separation column is a column in a diphenyl carbonate production facility and the method comprises adding a side feed stream originating from the diphenyl carbonate production facility to the downstream separation column; wherein the side feed stream comprises diphenyl carbonate and phenyl methyl carbonate.

14. The method of any one of the preceding claims, wherein the method further comprises directing one or both of the solvent recovery stream or the phenol recovery stream to a reactive distillation column in a diphenyl carbonate production facility.

15. The method of any one of the preceding claims, wherein the melt polymerizing comprises melt polymerizing bisphenol A and diphenyl carbonate.

## Patentansprüche

1. Verfahren zur Herstellung eines Schmelzpolycarbonats, umfassend:
Schmelzpolymerisieren eines Bisphenols und von Diphenylcarbonat, um Phenol und das Schmelzpolycarbonat zu bilden, in einer Polymerisationseinheit;
Entfernen eines Kopfstroms, der das Phenol in einer Gasphase umfasst, aus der Polymerisationseinheit;
Kombinieren des Kopfstroms mit einem Gasstrom, der ein Lösungsmittel in einer Gasphase umfasst, um einen kombinierten Gasstrom zu bilden; wobei der kombinierte Gasstrom einen Gefrierpunkt bei weniger als 3 mbar absolut, der niedriger als der Tripelpunkt von Phenol ist, aufweist; und
Kondensieren des kombinierten Gasstroms in einem Kondensator, um einen kondensierten Strom zu bilden, der des Phenol und das Lösungsmittel in einer flüssigen Phase umfasst.

2. Verfahren gemäß Anspruch 1, wobei das Lösungsmittel wenigstens eines von einem Gefrierpunkt von niedriger als oder gleich 40 °C oder niedriger oder gleich 35 °C bei 3 mbar absolut oder einen Dampfdruck bei 15 °C von niedriger als oder gleich 0,5 mbar aufweist.

3. Verfahren gemäß einem der vorstehenden Ansprüche, wobei das Lösungsmittel wenigstens eines von einem Arylalkylcarbonat, einem Dialkylcarbonat, Anisol und einem Alkylenglycol umfasst.

4. Verfahren gemäß einem der vorstehenden Ansprüche, wobei das Lösungsmittel wenigstens eines von Phenylmethylcarbonat, Anisol, Ethylenglycol und Propylenglycol umfasst.

5. Verfahren gemäß einem der vorstehenden Ansprüche, wobei der kombinierte Gasstrom 30 bis 90 Gew.-%, vorzugsweise 30 bis 70 Gew.-%, an dem Kopfstrom, bezogen auf das Gesamtgewicht des kombinierten Stroms, umfasst.

6. Verfahren gemäß einem der vorstehenden Ansprüche, wobei die einzigen Phasen des Lösungsmittels und des Phenols in dem Kondensator Flüssigkeits- und Dampfphasen sind.

7. Verfahren gemäß einem der vorstehenden Ansprüche, ferner umfassend
Trennen des kondensierten Stroms in einer Trennkolonne in einen Diphenylcarbonatgewinnungsstrom, einen Lösungsmittelgewinnungsstrom und einen Phenolgewinnungsstrom; oder
Trennen des kondensierten Stroms in der Trennkolonne in einen Bodenstrom und den Phenolgewinnungsstrom und ferner Trennen des Bodenstroms in einer nachgereihten Trennkolonne und Gewinnen des Lösungsmittelgewinnungsstroms aus der nachgereihten Trennkolonne.

8. Verfahren gemäß Anspruch 7, wobei die Trennkolonne einen oberen Abschnitt, einen mittleren Abschnitt und einen unteren Abschnitt umfasst; und das Verfahren Zuführen des kondensierten Stroms zwischen dem mittleren Abschnitt und dem oberen Abschnitt umfasst.

9. Verfahren gemäß einem der Ansprüche 7 bis 8, wobei das Trennen das Trennen des Diphenylcarbonatgewinnungsstroms von einem unteren Ende der Trennkolonne, des Lösungsmittelgewinnungsstroms von einer Seite der Trennkolonne und des Phenolgewinnungsstroms von einem oberen Ende der Trennkolonne umfasst.

10. Verfahren gemäß Anspruch 9, ferner umfassend Leiten des Lösungsmittelgewinnungsstrom zu einer Entspannungseinheit; Entnehmen eines entspannten Dampfstroms und eines Rückführstroms aus der Entspannungseinheit; und Leiten des Rückführstroms zurück in die Trennkolonne; wobei der entspannte Dampfstrom 70 bis 99 Gew.-% an dem Lösungsmittel, bezogen auf das Gesamtgewicht des entspannten Dampfstroms, umfasst.

11. Verfahren gemäß Anspruch 7, wobei das Verfahren Trennen des Bodenstroms von einem unteren Ende der Trennkolonne und des Phenolgewinnungsstroms von einem oberen Ende der Trennkolonne umfasst; und ferner Trennen des Bodenstroms in der nachgereihten Trennkolonne und Gewinnen des Lösungsmittelgewinnungsstroms aus der nachgereihten Trennkolonne umfasst.

12. Verfahren gemäß Anspruch 11, wobei das Gewinnen des Lösungsmittelgewinnungsstroms aus der nachgereihten Trennkolonne Entnehmen eines Kopfstroms aus der nachgereihten Trennkolonne; Leiten wenigstens eines Teils des Kopfstroms zu einer Entspannungseinheit; Entnehmen des Lösungsmittelgewinnungsstroms und eines Rückstroms aus der Entspannungseinheit; und Leiten des Rückstroms zu der nachgereihten Trennkolonne umfasst.

13. Verfahren gemäß einem der Ansprüche 7, 11 oder 12, wobei die nachgereihte Trennkolonne eine Kolonne in einer Diphenylcarbonat-Produktionsanlage ist und das Verfahren Zugeben eines Seiten-Zufuhrstroms, der aus der Diphenylcarbonat-Produktionsanlage stammt, zu der nachgereihten Trennkolonne umfasst; wobei der Seiten-Zufuhrstrom Diphenylcarbonat und Phenylmethylcarbonat umfasst.

14. Verfahren gemäß einem der vorstehenden Ansprüche, wobei das Verfahren ferner Leiten eines oder beider von dem Lösungsmittelgewinnungsstrom und dem Phenolgewinnungsstrom zu einer reaktiven Destillationskolonne in einer Diphenylcarbonat-Produktionsanlage umfasst.

15. Verfahren gemäß einem der vorstehenden Ansprüche, wobei die Schmelzepolymerisation Schmelzepolymerisieren von Bisphenol A und Diphenylcarbonat umfasst.

## Revendications

1. Procédé de fabrication d'un polycarbonate fondu, comprenant :
la polymérisation en fusion d'un bisphénol et de carbonate de diphényle pour former un phénol et le polycarbonate fondu dans une unité de polymérisation ;
l'élimination d'un flux de tête comprenant le phénol dans une phase gazeuse de l'unité de polymérisation ;
la combinaison du flux de tête avec un flux de gaz comprenant un solvant dans une phase gazeuse pour former un flux de gaz combiné ; le flux de gaz combiné ayant un point de congélation à moins de 3 mbar absolus qui est inférieur au point triple du phénol ; et
la condensation du flux de gaz combiné dans un condenseur pour former un flux condensé comprenant le phénol et le solvant dans une phase liquide.

2. Procédé selon la revendication 1, le solvant ayant au moins l'un parmi un point de congélation inférieur ou égal à 40 °C, ou inférieur ou égal à 35 °C à 3 mbar absolus ou une pression de vapeur à 15 °C inférieure ou égale à 0,5 mbar.

3. Procédé selon l'une quelconque des revendications précédentes, le solvant comprenant au moins l'un parmi un carbonate d'aryle et d'alkyle, un carbonate de dialkyle, l'anisole, ou un alkylèneglycol.

4. Procédé selon l'une quelconque des revendications précédentes, le solvant comprenant au moins l'un parmi le carbonate de phényle et de méthyle, l'anisole, l'éthylène glycol, ou le propylèneglycol.

5. Procédé selon l'une quelconque des revendications précédentes, le flux de gaz combiné comprenant 30 à 90 % en poids, préférablement, 30 à 70 % en poids du flux de tête sur la base du poids total du flux combiné.

6. Procédé selon l'une quelconque des revendications précédentes, les seules phases du solvant et du phénol dans le condenseur étant des phases liquides et vapeur.

7. Procédé selon l'une quelconque des revendications précédentes, comprenant en outre
la séparation du flux condensé dans une colonne de séparation en un flux de récupération de carbonate de diphényle, un flux de récupération de solvant, et un flux de récupération de phénol ; ou
la séparation du flux condensé dans la colonne de séparation en un flux de fond et le flux de récupération de phénol, et en outre la séparation du flux de fond dans une colonne de séparation en aval et la récupération du flux de récupération de solvant depuis la colonne de séparation en aval.

8. Procédé selon la revendication 7, la colonne de séparation comprenant une section supérieure, une section centrale, et une section inférieure ; et le procédé comprenant l'ajout du flux condensé entre la section centrale et la section supérieure.

9. Procédé selon l'une quelconque des revendications 7 à 8, la séparation comprenant la séparation du flux de récupération de carbonate de diphényle d'une extrémité de fond de la colonne de séparation, du flux de récupération du solvant d'un côté de la colonne de séparation, et du flux de récupération de phénol d'une extrémité sommitale de la colonne de séparation.

10. Procédé selon la revendication 9, comprenant en outre l'orientation du flux de récupération de solvant vers une unité de vaporisation instantanée ; le soutirage d'un flux de vapeur de vaporisation instantanée et flux de recyclage de l'unité de vaporisation spontanée ; et l'orientation du flux de recyclage de retour dans la colonne de séparation ; le flux de vapeur de vaporisation instantanée comprenant 70 à 99 % en poids du solvant sur la base du poids total du flux de vapeur de vaporisation instantanée.

11. Procédé selon la revendication 7, le procédé comprenant la séparation du flux de fond d'une extrémité de fond de la colonne de séparation et du flux de récupération de phénol d'une extrémité sommitale de la colonne de séparation ; et en outre la séparation du flux de fond dans la colonne de séparation en aval et la récupération du flux de récupération de solvant de la colonne de séparation en aval.

12. Procédé selon la revendication 11, la récupération du flux de récupération de solvant de la colonne de séparation en aval comprenant le soutirage d'un flux sommital de la colonne de séparation en aval ; l'orientation d'au moins une portion du flux sommital vers une unité de vaporisation instantanée ; le soutirage du flux de récupération de solvant et d'un flux de retour de l'unité de vaporisation instantanée ; et l'orientation du flux de retour vers la colonne de séparation en aval.

13. Procédé selon l'une quelconque des revendications 7, 11 ou 12, la colonne de séparation en aval étant une colonne dans une installation de production de carbonate de diphényle et le procédé comprenant l'ajout d'un flux d'alimentation latérale provenant de l'installation de production de carbonate de diphényle à la colonne de séparation en aval ; le flux d'alimentation latérale comprenant du carbonate de diphényle et du carbonate de phényle de méthyle.

14. Procédé selon l'une quelconque des revendications précédentes, le procédé comprenant en outre l'orientation de l'un ou des deux parmi le flux de récupération de solvant ou le flux de récupération de phénol vers une colonne de distillation réactive dans une installation de production de carbonate de diphényle.

15. Procédé selon l'une quelconque des revendications précédentes, la polymérisation en fusion comprenant la polymérisation en fusion de bisphénol A et de carbonate de diphényle.
